(19) Europäisches Patentamt
European Patent Office
Office européen des brevets

(11) Numéro de publication: **0 241 338 B1**

# (12) FASCICULE DE BREVET EUROPEEN

(45) Date de publication du fascicule du brevet: **05.09.90**

(51) Int. Cl.⁵: **C08F 20/36**, C09K 19/38

(21) Numéro de dépôt: **87400620.8**

(22) Date de dépôt: **19.03.87**

(54) **Polymères mesomorphes à chaines latérales à forte anisotropie diélectrique et leur procédé de fabrication.**

(30) Priorité: **25.03.86 FR 8604285**

(43) Date de publication de la demande:
**14.10.87 Bulletin 87/42**

(45) Mention de la délivrance du brevet:
**05.09.90 Bulletin 90/36**

(84) Etats contractants désignés:
**DE GB IT NL**

(56) Documents cités:
**EP-A- 0 007 574**
**GB-A- 2 002 767**

(73) Titulaire: **THOMSON-CSF, 51, Esplanade du Général de Gaulle, F-92800 Puteaux(FR)**

(72) Inventeur: **Le Barny, Pierre, c/o THOMSON-CSF, SCPI, 19, avenue de Messine, F-75008 Paris(FR)**
Inventeur: **Ravaux, Gilles, c/o THOMSON-CSF, SCPI, 19, avenue de Messine, F-75008 Paris(FR)**
Inventeur: **Dubois, Jean-Claude, c/o THOMSON-CSF, SCPI, 19, avenue de Messine, F-75008 Paris(FR)**

(74) Mandataire: **Lepercque, Jean et al, THOMSON-CSF SCPI, F-92045 PARIS LA DEFENSE CEDEX 67(FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

L'invention concerne une famille d'homopolymères à chaînes latérales du type polyacrylate dérivé du 3 fluoro 4 cyanophénol et présentant une mésophase de type nématique ou smectique A.

Actuellement on envisage de plus en plus d'utiliser des matériaux polymères dans des dispositifs relevant du domaine de l'optique non linéaire ou du domaine de l'affichage. L'utilisation de ces polymères dans des dispositifs d'affichage est soumise à deux conditions : ils doivent posséder une anisotropie diélectrique élevée et également une mésophase nématique ou smectique A. Il n'existe apparemment pas de polymères possédant à la fois ces deux caractéristiques.

Pour pallier ces inconvénients, l'invention propose une famille d'homopolymères possédant à la fois une anisotropie élevée (de l'ordre de 20 à température ambiante) et une mésophase nématique ou smectique A. La combinaison de ces deux caractéristiques est obtenue par l'introduction, dans la molécule de base du polymère, d'un atome de fluor en position ortho d'un groupe cyano.

L'invention a donc pour objet un polymère mésomorphe à chaîne latérale à forte anisotropie diélectrique, caractérisé en ce qu'il répond à la formule chimique générale suivante :

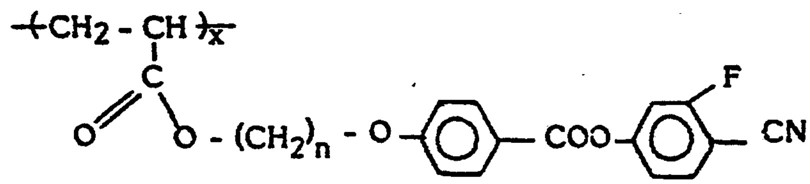

x indiquant le degré de polymérisation et $4 \leq n \leq 15$.

L'invention a aussi pour objet un procédé de fabrication d'un polymère mésomorphe répondant à la formule chimique ci-dessus, caractérisé en ce qu'il comporte, dans le cas où $5 \leq n \leq 15$, les étapes suivantes :

-lère étape : obtention de l'ω-bromoalcanol par réduction de l'acide ω-bromoalcanoïque correspondant au moyen de l'hydrure de lithium et d'aluminium dans l'éther,
- 2ème étape : synthèse de l'acide 4(ω-hydroxyalkyloxybenzoïque par action de l'ω-bromoalcanol sur l'acide 4 hydroxybenzoïque en milieu potasse hydroalcoolique,
- 3ème étape : obtention de l'acide 4 (acryloyloxyalkyloxy)benzoïque par estérification de l'acide obtenu à l'issue de la deuxième étape au moyen de l'acide acrylique,
- 4ème étape : synthèse du chlorure de 4-(acryloyloxyalkyloxy) benzoyle au moyen du chlorure d'oxalyle avec le diméthyl formamide comme catalyseur,
- 5ème étape : obtention du 4-[4'-(acryloyloxyalkyloxy)benzoyloxy]-1- cyano-2-fluorobenzène par action du chlorure obtenu à l'issue de la quatrième étape sur le 4-cyano-3-fluorophénol dans le tétrahydrofuranne en présence de triéthylamine,
- 6ème étape : polymérisation du monomère obtenu à l'issue de la cinquième étape.

L'invention a encore pour objet un procédé de fabrication d'un polymère mésomorphe répondant à la même formule chimique caractérisé en ce qu'il comporte, dans le cas où n = 4, les étapes suivantes:

- 1ère étape : protection de la fonction acide de l'acide 4-hydroxybenzoïque par action du méthanol,
- 2ème étape : éthérification du produit obtenu à l'issue de la première étape par action du 1,4-dibromobutane,
- 3ème étape : déprotection de la fonction acide du produit obtenue à l'issue de la seconde étape par réaction en milieu potasse alcoolique puis en milieu acide,
- 4ème étape : estérification du produit obtenu à l'issue de la troisième étape par l'acrylate de lithium dans l'hexaméthylphosphoramide,
- 5ème étape : synthèse du chlorure de 4-(acryloyloxybutyloxy) benzoyle par action du chlorure d'oxalyle sur le produit obtenu à l'issue de la quatrième étape et avec le diméthyl formamide comme catalyseur,
- 6ème étape : obtention du 4-[4'-(acryloyloxyalkyloxy)benzoyloxy] 1-cyano 2-fluorobenzène par action du chlorure obtenu à l'issue de la cinquième étape sur le 4 cyano 3 fluorophénol dans le tétrahydrofuranne en présence de triéthylamine,
- 7ème étape : polymérisation du monomère obtenu à l'issue de la sixième étape.

L'invention sera mieux comprise et d'autres avantages apparaîtront au moyen de la description qui va suivre et des figures annexées parmi lesquelles :

- la figure 1 est un tableau explicatif de conditions de polymérisation,
- la figure 2 est un tableau présentant des transitions de phases de polymères selon l'invention,
- la figure 3 est un diagramme représentant l'évolution de la constante diélectrique d'un polymère selon l'invention,

- la figure 4 est un diagramme représentant l'évolution de la constante diélectrique d'un polymère selon l'art connu.

La description qui va suivre portera sur le procédé général de synthèse des polymères selon l'invention ainsi que sur leurs caractérisations physiques.

PROCEDE GENERAL DE SYNTHESE.

Les polymères selon l'invention sont obtenus généralement en six étapes à partir des produits commerciaux suivants : l'acide ω-bromoalcanoïque, l'acide 4-hydroxybenzoïque, l'acide acrylique et le 3-fluoro-4-cyanophénol dont la synthèse est décrite par S.M. KELLY dans la revue Helvetica Chimica Acta, tome 67, 1984, pp. 1572-1579.

Réaction 1 : Obtention de l'ω-bromoalcanol.

Cet alcool est obtenu par réduction de l'acide ω-bromoalcanoïque correspondant au moyen de l'hydrure de lithium et d'aluminium dans l'éther.

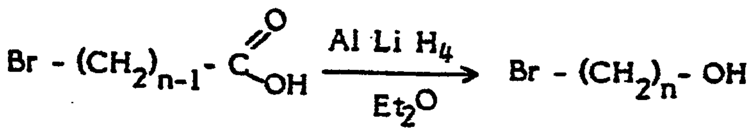


Réaction 2 : Synthèse de l'acide 4(ω-hydroxyalkyloxy) benzoïque.

Cet acide résulte de l'action de l'ω-bromoalcanol obtenu à l'issue de la réaction 1 sur l'acide 4-hydroxybenzoïque en milieu potasse hydroalcoolique.

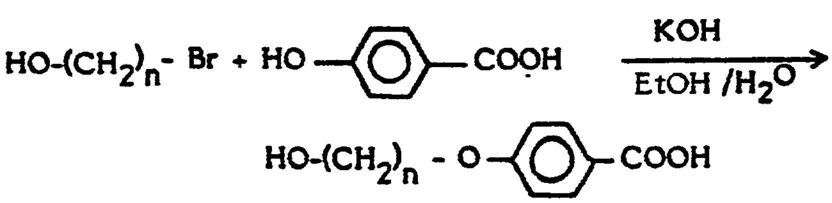


Réaction 3 : Obtention de l'acide 4(acryloyloxyalkyloxy) benzoïque.

L'estérification de l'acide 4(ω-hydroxyalkyloxy) benzoïque, obtenu à l'issue de la réaction 2, au moyen de l'acide acrylique conduit à l'acide 4(acryloyloxyalkyloxy) benzoïque. Cette estérification se fait en utilisant le benzène comme solvant et l'acide paratoluène sulfonique (APTS) comme catalyseur. Au cours de la réaction, l'hydroquinone est utilisée comme inhibiteur de polymérisation.

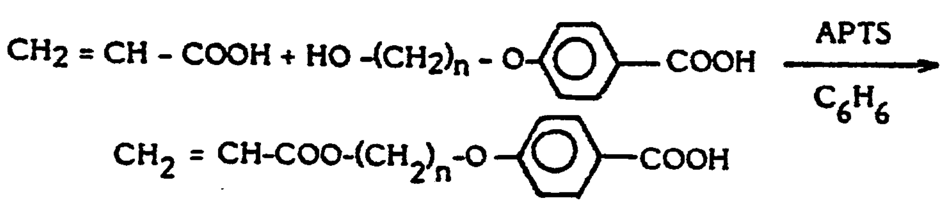


Réaction 4 : Synthèse du chlorure de 4(acryloyloxyalkyloxy) benzoyle.

Le passage de l'acide obtenu à l'issue de la réaction 3 au chlorure d'acide se fait à froid au moyen du chlorure d'oxalyle en utilisant le diméthyl formamide (DMF) comme catalyseur.

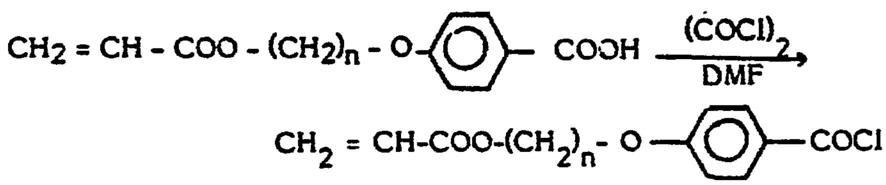


L'utilisation du chlorure d'oxalyle à la place du chlorure de thionyle pour préparer le chlorure d'acide désiré permet de préserver la fonction acrylate.

<u>Réaction 5</u> : Obtention du 4-[4'–(acryloyloxyalkyloxy) benzoyloxy]-1-cyano-2-fluorobenzène.

Cet ester est synthétisé par action du chlorure de 4(acryloyloxyalkyloxy) benzoyle, obtenu à l'issue de la réaction 4, sur le 4-cyano-3-fluorophénol dans le tétrahydrofuranne (THF) en présence de triéthylamine ($Et_3N$).

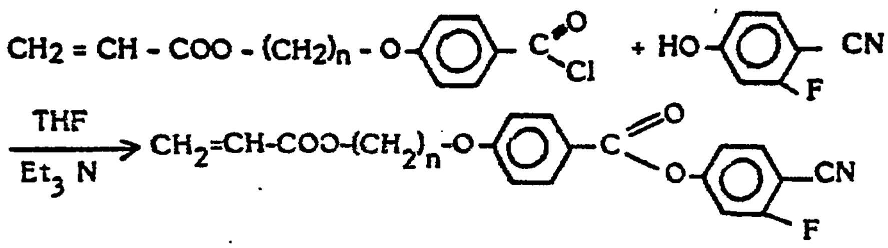


Il faut noter que pour n = 4 la réaction 2 n'est pas possible. En effet, en milieu basique le 4-bromol-1-butanol se cyclise selon la réaction suivante :

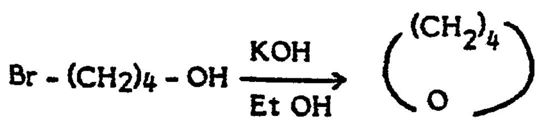


Pour obtenir l'acide 4-[4'-acryloyloxybutyloxy] benzoïque (c'est-à-dire le produit final correspondant à la réaction 3 dans le procédé général de synthèse), on doit adopter le schéma réactionnel suivant.

. protection de la fonction acide de l'acide 4 hydroxybenzoïque.

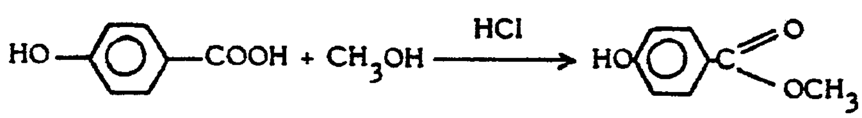


. éthérification au moyen du 1,4-dibromobutane.

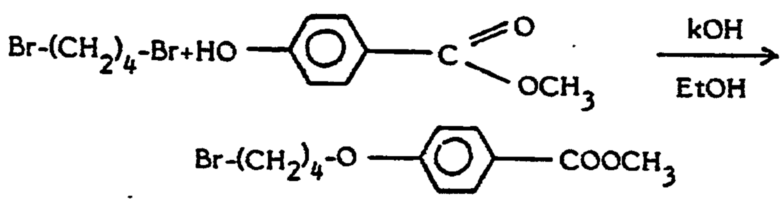


. déprotection de la fonction acide.

Cette réaction s'effectue d'abord en milieu potasse alcoolique puis en milieu acide.

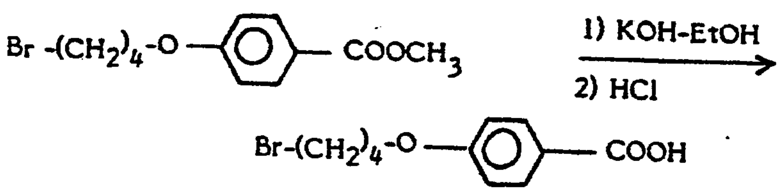


. estérification avec l'acrylate de lithium dans l'hexaméthylphosphoramide (HMPA).

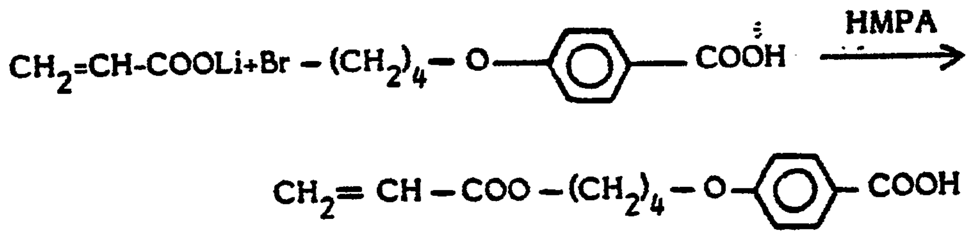


On voit donc que pour n = 4, les réactions 1 à 3 du procédé général de synthèse sont remplacées par les quatre réactions ci-dessus.

Réaction 6 : Polymérisation des monomères.

La polymérisation peut être effectuée sous vide en utilisant l'azobisisobutyronitrile comme amorceur radicalaire.

Les réactions 2 et 3 sont décrites dans la demande de brevet DE 3 211 400. Les autres réactions prises isolément sont classiques.

Les polymères obtenus répondent aux dénominations suivantes:

poly[4(4'-acryloyloxybutyloxy benzoyloxy)-4"-cyano-3-fluorophényl]
poly[4(4'-acryloyloxypentyloxy benzoyloxy)-4"-cyano-3-fluorophényl]
poly[4(4'-acryloyloxyhexyloxy benzoyloxy)-4"-cyano-3-fluorophényl]
poly[4(4'-acryloyloxyheptyloxy benzoyloxy)-4"-cyano-3-fluorophényl]
poly[4(4'-acryloyloxyoctyloxy benzoyloxy)-4"-cyano-3-fluorophényl]
poly[4(4'-acryloyloxynonyloxy benzoyloxy)-4"-cyano-3-fluorophényl]
poly[4(4'-acryloyloxydécyloxy benzoyloxy)-4"-cyano-3-fluorophényl]
poly[4(4'-acryloyloxyundécyloxy benzoyloxy)-4"-cyano-3-fluorophényl]
poly[4(4'-acryloyloxydodécyloxy benzoyloxy)-4"-cyano-3-fluorophényl]
poly[4(4'-acryloyloxytridécyloxy benzoyloxy)-4"-cyano-3-fluorophényl]
poly[4(4'-acryloyloxytétradécyloxy benzoyloxy)-4"-cyano-3-fluorophényl]
poly[4(4'-acryloyloxypentadécyloxy benzoyloxy)-4"-cyano-3-fluorophényl]

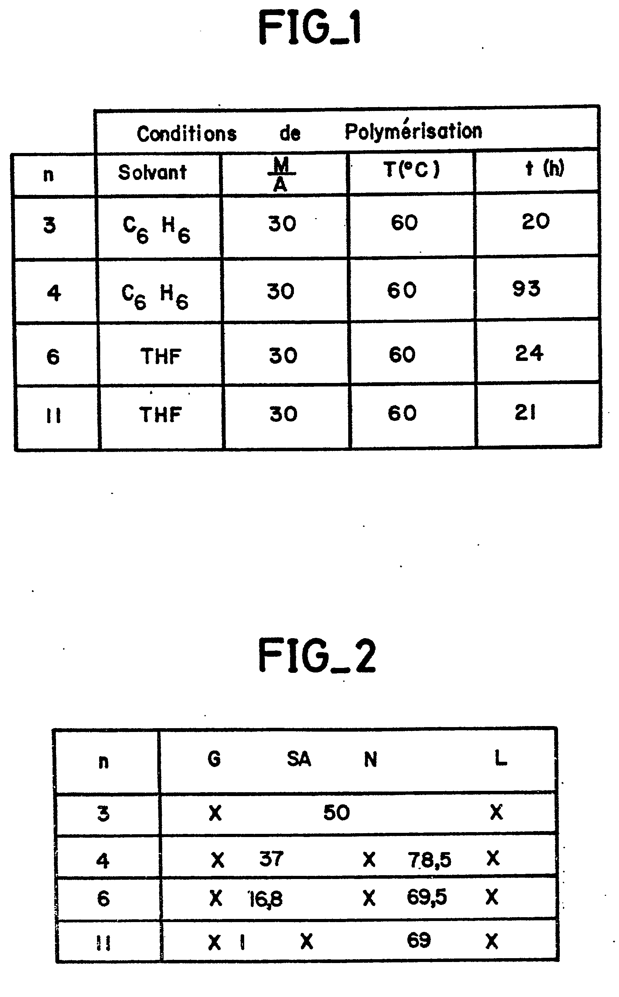

A titre d'exemple, quelques conditions de polymérisation sont rassemblées dans le tableau de la figure 1. On a indiqué dans ce tableau la nature du solvant utilisé, le rapport M/A (M étant le nombre de moles du monomère et A le nombre de moles d'amorceur radicalaire), la température T en degrés Celsius et le temps t en heures. Les polymérisations ont été effectuées pour quelques valeurs de n indiquées dans le tableau de la figure 1.

PROPRIETES PHYSIQUES DES POLYMERES.

Afin de préciser leurs propriétés mésomorphes, les polymères ont été étudiés par analyse enthalpique différentielle, microscopie optique et diffraction des rayons X.

A titre d'exemple non limitatif, le tableau de la figure 2 regroupe les résultats obtenus pour différentes valeurs de n. Dans ce tableau, les lettres G, SA, N et L désignent respectivement les phases vitreuse, smectique A, nématique et liquide. Les croix situées sous ces lettres indiquent la transition d'une phase à une autre phase. Les températures de transition indiquées dans ce tableau sont exprimées en degrés Celsius. On remarque que les propriétés mésomorphes n'apparaissent que pour n ≥ 4. En effet, pour n = 3 il n'existe qu'une transition (à 50°C) directement de la phase vitreuse à la phase liquide. Les autres polymères selon l'invention comportent une phase nématique ou smectique A. La phase nématique s'étend sur 41,5°C pour n = 4, sur 52,7°C pour n = 6. La phase smectique A s'étend sur 68°C pour n = 11.

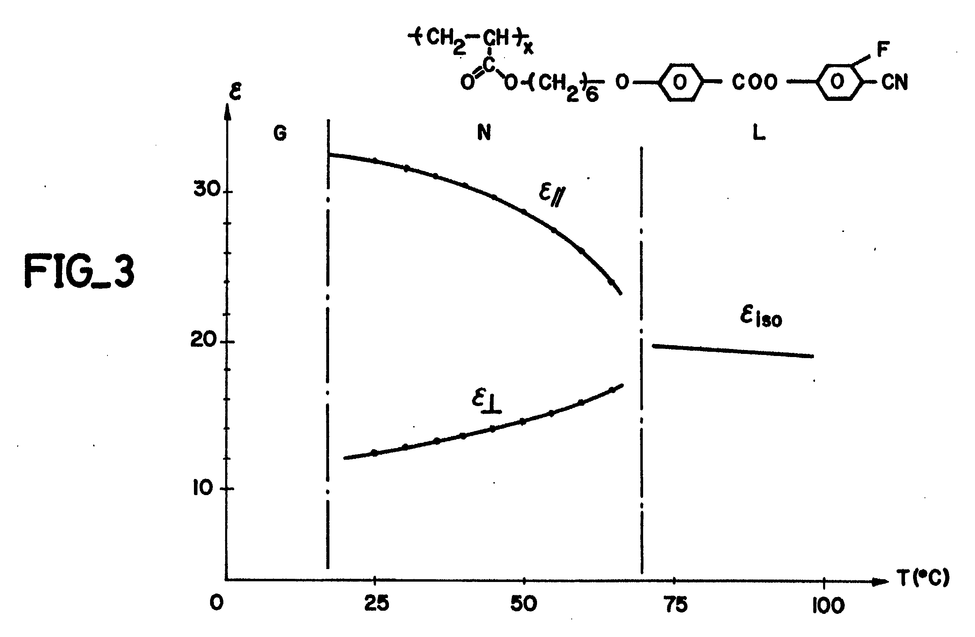

Les propriétés diélectriques des polymères selon l'invention sont particulièrement intéressantes. A titre d'exemple, la figure 3 montre la valeur des constantes diélectriques mesurées en fonction de la température, pour un champ électrique parallèle à l'axe optique ($\varepsilon_{\parallel}$) et pour un champ électrique perpendiculaire à l'axe optique ($\varepsilon_{\perp}$). Les courbes de la figure 3 ont été tracées pour n = 6. Le diagramme de la figure 3 est divisé en trois zones par des droites élevées à partir de l'axe des températures. Ces zones correspondent à des phases différentes du polymère dont les températures de transition sont répertoriées dans le tableau de la figure 2. On constate, dans la phase nématique, l'existence de deux courbes convergentes lorsque la température augmente. L'anisotropie diélectrique $\Delta\varepsilon = \varepsilon_{\parallel} - \varepsilon_{\perp}$ est positive et sa valeur à 25°C est de 19,6. Dans la phase liquide, il n'existe plus bien sûr qu'une seule courbe correspondant à la constante diélectrique isotrope.

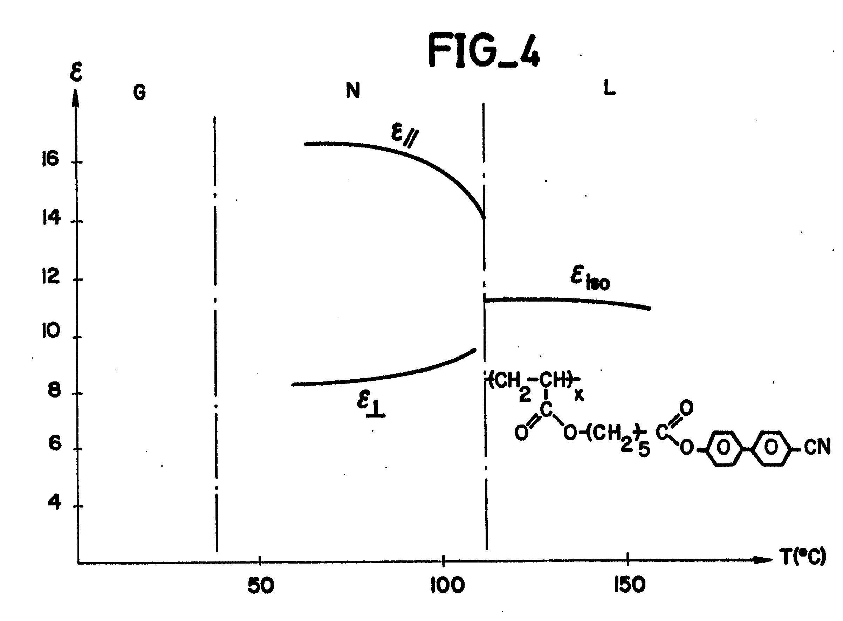

Le diagramme de la figure 4 représente l'évolution de la constante diéelctrique d'un polymère nématique de l'art connu en fonction de la température. Il s'agit d'un polyacrylate dérivé du cyanobiphényle dont la formule chimique est indiquée sur la figure. Les courbes ont été tracées à partir des valeurs ε (γ) extrapolées à fréquence nulle. Ce polymère comprend trois phases : vitreuse, nématique et liquide. La plage nématique de ce composant est grande (environ 70,3°C). L'anisotropie diélectrique la plus élevée

que l'on puisse relever sur ce diagramme est de l'ordre de 8,2 à 70°C soit 2,4 fois plus faible que la valeur signalée plus haut pour le composé selon l'invention (19,6 à 25°C).

Il apparaît que l'introduction d'un atome de fluor en position ortho du groupe cyano diminue le degré d'association entre les chaînes latérales voisines des macromolécules et augmente par conséquent l'anisotropie diélectrique. Ceci explique la valeur de 19,6 mesurée à 25°C.

Les polymères selon l'invention ayant une température de transition vitreuse inférieure à la température ambiante (n = 6 ou 11 par exemple) pourront être utilisés avec intérêt dans des dispositifs d'affichage du type nématique (encore appelé "guest-host" selon la terminologie anglo-saxonne) ou smectique A. En effet, leur forte anisotropie diélectrique permet la diminution de la tension de seuil du dispositif puisque cette dernière est inversement proportionnelle à

$$\sqrt{\Delta\varepsilon}.$$

Les polymères selon l'invention ayant une température de transition vitreuse supérieure à la température ambiante (n = 4 par exemple) ont une application possible en optique non linéaire et plus précisément en génération de seconde harmonique. Ils peuvent en effet servir de matrice d'orientation à des petites molécules ayant une forte hyperpolarisabilité du second ordre et, grâce à leur degré d'association moindre dans les chaînes latérales, conduire à des systèmes non centro-symétriques après orientation sous champ électrique plus aisément que les polymères cyanés.

**Revendications**

1. Polymère mésomorphe à chaîne latérale à forte anisotropie diélectrique, caractérisé en ce qu'il répond à la formule chimique générale suivante:

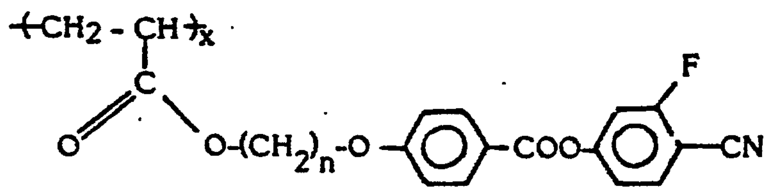

x indiquant le degré de polymérisation et 4 ≦ n ≦ 15.

2. Procédé de fabrication d'un polymère mésomorphe selon la revendication 1, caractérisé en ce qu'il comporte, dans le cas où 5 ≦ n ≦ 15, les étapes suivantes :

- 1ère étape : obtention de l'ω-bromoalcanol par réduction de l'acide ω-bromoalcanoïque correspondant au moyen de l'hydrure de lithium et d'aluminium dans l'éther,
- 2ème étape : synthèse de l'acide 4(ω-hydroxyalkyloxybenzoïque par action de l'ω-bromoalcanol sur l'acide 4-hydroxybenzoïque en milieu potasse hydroalcoolique,
- 3ème étape : obtention de l'acide 4-(acryloyloxyalkyloxy)benzoïque par estérification de l'acide obtenu à l'issue de la deuxième étape au moyen de l'acide acrylique,
- 4ème étape : synthèse du chlorure de 4-(acryloyloxyalkyloxy) benzoyle au moyen du chlorure d'oxalyle avec le diméthyl formamide comme catalyseur,
- 5ème étape : obtention du 4-[4'-(acryloyloxyalkyloxy)benzoyloxy]1-cyano-2-fluorobenzène par action du chlorure obtenu à l'issue de la quatrième étape sur le 4-cyano-3-fluorophénol dans le tétrahydrofuranne en présence de triéthylamine,
- 6ème étape : polymérisation du monomère obtenu à l'issue de la cinquième étape.

3. Procédé de fabrication d'un polymère mésomorphe selon la revendication 1, caractérisé en ce qu'il comporte, dans le cas où n = 4, les étapes suivantes :

- 1ère étape : protection de la fonction acide de l'acide 4 hydroxybenzoïque par action du méthanol,
- 2ème étape : éthérification du produit obtenu à l'issue de la première étape par action du 1,4-dibromobutane,
- 3ème étape : déprotection de la fonction acide du produit obtenu à l'issue de la seconde étape par réaction en milieu potasse alcoolique puis en milieu acide,
- 4ème étape : estérification du produit obtenu à l'issue de la troisième étape par l'acrylate de lithium dans l'hexaméthylphosphoramide,
- 5ème étape : synthèse du chlorure de 4-(acryloyloxybutyloxy) benzoyle par action du chlorure d'oxalyle sur le produit obtenu à l'issue de la quatrième étape et avec le diméthyl formamide comme catalyseur,
- 6ème étape : obtention du 4-[4'-(acryloyloxyalkyloxy)benzoyloxy]1-cyano-2-fluorobenzène par action du chlorure obtenu à l'issue de la cinquième étape sur le 4 cyano 3 fluorophénol dans le tétrahydrofuranne en présence de triéthylamine,
- 7ème étape : polymérisation du monomère obtenu à l'issue de la sixième étape.

## Patentansprüche

1. Mesomorphes Polymer mit Seitenkette und mit starker dielektrischer Anisotropie, dadurch gekennzeichnet, daß es der folgenden allgemeinen chemischen Formel entspricht:

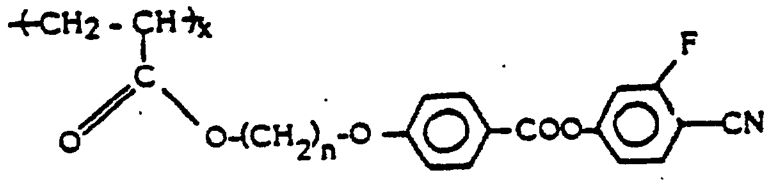

wobei x den Polymerisationsgrad angibt und $4 \leq n \leq 15$.

2. Verfahren zur Herstellung eines mesomorphen Polymers nach Anspruch 1, dadurch gekennzeichnet, daß es für $5 \leq n \leq 15$ die folgenden Stufen umfaßt:
- erste Stufe: Herstellung von ω-Bromalkanol durch Reduktion der entsprechenden ω-Bromalkansäure mit Lithiumaluminiumhydrid in Äther,
- zweite Stufe: Synthese der 4ω-Hydroxyalkyloxybenzoesäure durch Umsetzung von ω-Bromalkanol mit 4-Hydroxybenzoesäure in wäßrig-alkoholischem Kaliumhydroxid-Milieu,
- dritte Stufe: Herstellung der 4-(Acryloyloxyalkyloxy)benzoesäure durch Veresterung der in der zweiten Stufe erhaltenen Säure mit Acrylsäure,
- vierte Stufe: Synthese des 4-(Acryloyloxyalkyloxy)benzoylchlorids mit Oxalylchlorid, mit Dimethylformamid als Katalysator,
- fünfte Stufe: Herstellung von 4-[4'-(Acryloyloxyalkyloxy)benzoyloxy]1-cyano-2-fluorbenzol durch Umsetzung des in der vierten Stufe erhaltenen Chlorids mit 4-Cyano-3-fuorphenol in Tetrahydrofuran in Gegenwart von Triethylamin, und
- sechste Stufe: Polymerisation des in der fünften Stufe erhaltenen Monomers.

3. Verfahren zur Herstellung eines mesomorphen Polymers nach Anspruch 1, dadurch gekennzeichnet, daß es für $n = 4$ die folgenden Stufen umfaßt:
- erste Stufe: Schützen der Säurefunktion der 4-Hydroxybenzoesäure duch Umsetzung mit Methanol,
- zweite Stufe: Verätherung des in der ersten Stufe erhaltenen Produkts durch Umsetzung mit 1,4-Dibrombutan,
- dritte Stufe: Entfernung der Schutzgruppe aus der Säurefunktion des in der zweiten Stufe erhaltenen Produkts durch Reaktion in einem alkoholischen Kaliumhydroxid-Milieu und dann in einem Säure-Milieu,
- vierte Stufe: Veresterung des in der dritten Stufe erhaltenen Produkts mit Lithiumacrylat in Hexamethylphosphoramid,
- fünfte Stufe: Synthese des 4-(Acryloyloxybutyloxy)benzoylchlorids durch Umsetzung von Oxalylchlorid mit dem in der vierten Stufe erhaltenen Produkt und mit Dimethylformamid als Katalysator,
- sechste Stufe: Herstellung von 4-[4'-(Acryloyloxyalkyloxy)benzoyloxy]1-cyano-2-fluorbenzol durch Umsetzung des in der fünften Stufe erhaltenen Chlorids mit 4-Cyano-3-fluorphenol in Tetrahydrofuran in Gegenwart von Triäthylamin, und
- siebte Stufe: Polymerisation des in der sechsten Stufe erhaltenen Monomers.

## Claims

1. A mesomorphous polymer with side chain exhibiting a strong dielectric anisotropy, characterized in that it corresponds to the following general chemical formula:

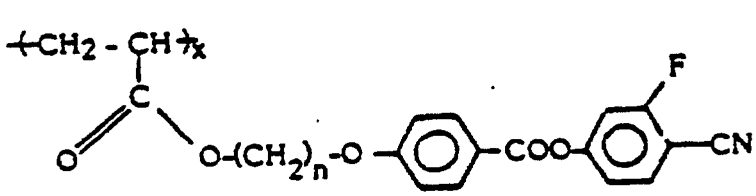

where x represents the degree of polymerisation and $4 \leq n \leq 15$.

2. A method of manufacturing a mesomorphous polymer according to claim 1, characterized in that, in the case of $5 \leq n \leq 15$, it comprises the following steps:
- first step: preparation of ω-bromoalcanol by reduction of the corresponding ω-bromoalcanoic acid with lithiumaluminum hydride in ether,
- second step: synthesis of the 4-ω-hydroxyalkyloxybenzoic acid by reacting ω-bromoalcanol with 4-hydroxybenzoic acid in a hydroalcoholic potash lye medium,

– third step: preparation of 4-(acryloyloxyalkyloxy)benzoic acid by esterification of the acid obtained in the second step by means of acrylic acid,
– fourth step: synthesis of the 4-(acryloyloxyalkyloxy)benzoyl chloride by means of oxalyl chloride with dimethyl formamide as catalyst,
– fifth step: preparation of 4-[4'-(acryloyloxyalkyloxy)benzoyloxy]-1-cyano-2-fluorobenzene by reacting the chloride obtained in the fourth step with 4-cyano-3-fluorophenol in tetrahydrofuran in the presence of triethylamine, and
– sixth step: polymerisation of the monomer obtained in the fifth step.

3. A process of manufacturing a mesomorphous polymer according to claim 1, characterized in that for n = 4 it comprises the following steps:
– first step: protecting the acid function of the 4-hydroxybenzoic acid by reacting it with methanol,
– second step: etherification of the product obtained in the first step by reacting it with 1,4-dibromobutane,
– third step: removing the protecting group from the acid function of the product obtained in the second step by a reaction in an alcoholic potash lye medium and then in an acid medium,
– fourth step: esterification of the product obtained in the third step with lithium acrylate in hexamethylphosphoramide,
– fifth step: synthesis of the 4-(acryloyloxybutyloxy)benzoyl chloride by reacting of the oxalyl chloride with the product obtained in the fourth step using dimethylformamide as a catalyst,
– sixth step: preparation of the 4-[4'-(acryloyloxyalkyloxy)benzoyloxy]1-cyano-2-fluorobenzene by reacting the chloride obtained in the fifth step with the 4-cyano-3-fluorophenol in tetrahydrofuran in the presence of triethylamine, and
– seventh step: polymerisation of the monomer obtained in the sixth step.

# FIG_1

| | Conditions de Polymérisation | | | |
|---|---|---|---|---|
| n | Solvant | M/A | T(°C) | t (h) |
| 3 | $C_6H_6$ | 30 | 60 | 20 |
| 4 | $C_6H_6$ | 30 | 60 | 93 |
| 6 | THF | 30 | 60 | 24 |
| 11 | THF | 30 | 60 | 21 |

# FIG_2

| n | G | | SA | | N | | L |
|---|---|---|---|---|---|---|---|
| 3 | X | | | 50 | | | X |
| 4 | X | 37 | | | X | 78,5 | X |
| 6 | X | 16,8 | | | X | 69,5 | X |
| 11 | X | I | X | | | 69 | X |

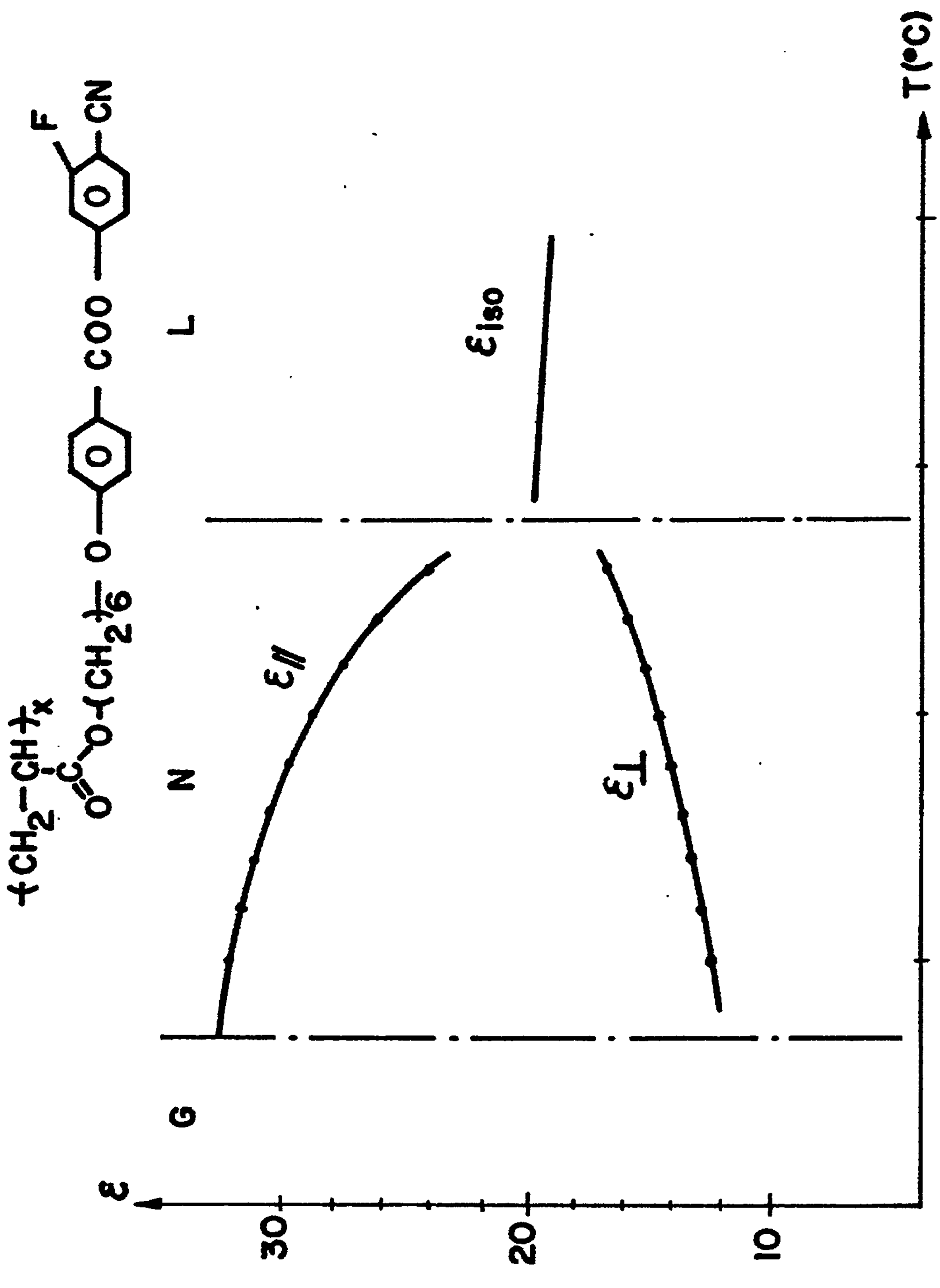


FIG_3

FIG_4